# EUROPEAN PATENT APPLICATION

(11) **EP 4 424 817 A1**
(43) Date of publication of application: **04.09.2024**
(21) Application number: 22886975.6
(22) Date of filing: 25.10.2022
(51) Int. Cl.: C12N 5/071, C12N 5/10, C12Q 1/02, G01N 33/15, G01N 33/50

(54) **METHOD FOR PRODUCING INTESTINAL ENDOCRINE CELLS, PLURIPOTENT STEM CELL-DERIVED INTESTINAL ENDOCRINE CELLS, MEDIUM OR MEDIUM KIT, AND USE OF THE SAME**

(30) Priority: 25.10.2021 JP 2021173827
(71) Applicant: FUJIFILM Corporation, Tokyo 106-8620 (JP)
(72) Inventor: YAMAZAKI, Nao, Ashigarakami-gun, Kanagawa 258-8577 (JP); MIMA, Shinji, Ashigarakami-gun, Kanagawa 258-8577 (JP); IMAKURA, Yuki, Ashigarakami-gun, Kanagawa 258-8577 (JP)
(74) Representative: Hoffmann Eitle
(86) International application number: PCT/JP2022/039602
(87) International publication number: WO 2023/074648

(57) **Abstract**

An object of the present invention is to provide a method of producing an enteroendocrine cell by using an intestinal stem cell induced from a pluripotent stem cell, a method of analyzing a disease mechanism, an evaluation method for a test substance, a screening method for a therapeutic drug for a digestive system disease, an enteroendocrine cell derived from a pluripotent stem cell, a culture medium or a culture medium kit, and a kit for producing an enteroendocrine cell. According to the present invention, there is provided a method of producing an enteroendocrine cell, the method including an intestinal stem cell or an intestinal epithelial cell, which is induced from a pluripotent stem cell, by using a culture medium that substantially does not contain a ligand of an epidermal growth factor but contains a cAMP activator and a DNA methyltransferase inhibitor.

## Description

### Technical Field

The present invention relates to a method of producing an enteroendocrine cell by culturing an intestinal stem cell or an intestinal epithelial cell. The present invention further relates to a method of analyzing a disease mechanism, an evaluation method for a test substance, and a screening method for a therapeutic drug for a digestive system disease, which use the enteroendocrine cells produced by the method according to the present invention. The present invention further relates to an enteroendocrine cell derived from a pluripotent stem cell. In addition, the present invention relates to a culture medium or a culture medium kit, and a kit for producing an enteroendocrine cell.

### Background Art

An intestinal endocrine system controls the response of a body to nutrients by secreting various hormones to finely adjust various physiological reactions in the body. The enteroendocrine cell (hereinafter, also referred to as EEC) forms an endocrine system largest in a body and thus plays an important role in diseases such as a gastrointestinal disorder, diabetes, and obesity. The enteroendocrine cells are individually dispersed along the crypt-villus axis throughout the entire intestinal epithelium; however, they are present in vivo at only a very small proportion (-1%).

An important function of the enteroendocrine cell is to sense the contents in the lumen, particularly nutrients, and to secrete various hormones (such as GLP-1) that regulate food intake, energy homeostasis, and glucose tolerance. In addition, since the enteroendocrine cell secretes hormones such as GLP-1, PYY, and GLP-2, it has been suggested that the enteroendocrine cell plays an important role in gastric bypass surgery. As a result, the enteroendocrine cell is a therapeutic target for diabetes and obesity. Further, it has also been reported that the enteroendocrine cell has an immune regulation function in innate immunity. The enteroendocrine cell expresses a functional Toll-like receptor (TLR) and directly responds to metabolites that are generated by symbiotic bacteria. In addition, it has also been suggested that the enteroendocrine cell may directly adjust the function of immune cells through a fluctuation in the number of cells and a change in hormone secretion during intestinal inflammation. The enteroendocrine cell is conceived to play an important role in gastrointestinal diseases such as metabolic diseases (diabetes, obesity, and the like), irritable bowel syndrome, infectious enteritis, and inflammatory bowel disease. As a result, there is a great interest in enteroendocrine cells for the investigation and development of disease intervention.

Patent Document 1 discloses a method for obtaining a population of enteroendocrine cells from a mammalian postnatal cell population, the method including treating a mammalian postnatal cell population with a plurality of small molecules that up-regulate CHGA to differentiate postnatal cells. In addition, Patent Document 2 discloses a method of producing an intestinal organoid, which includes a step of differentiating pluripotent stem cells into endoderm-like cells, a step of differentiating the obtained endoderm-like cells into intestinal stem cell-like cells, a step of culturing the obtained intestinal stem cell-like cells in a presence of an epithelial growth factor, a fibroblast growth factor, a TGFβ receptor inhibitor, a GSK-3β inhibitor, and a ROCK inhibitor, a step of culturing the obtained cells to form spheroids, and a step of differentiating the formed spheroids to form an intestinal organoid.

### Prior Art Documents

### Patent Documents

Patent Document 1: WO2017/120543A
Patent Document 2: WO2020/091020A

### SUMMARY OF THE INVENTION

There is a need for systems of culture and evaluation for in vitro enteroendocrine cells to enable the investigation of metabolic and digestive diseases. There is also a need for such a mechanism and compound search in which these systems of culture and evaluation for in vitro enteroendocrine cells are used to not only carry out pathological analysis and a therapeutic method but also regulate the function of enteroendocrine cells, thereby acquiring a specific enteroendocrine cell subtype.

For the effective use of enteroendocrine cells, it is necessary to carry out a detailed analysis of the enteroendocrine cells and the elucidation of the differentiation mechanism thereof; however, the enteroendocrine cells are present in vivo at only a very small proportion (-1%).

Patent Document 1 reports a production method for an enteroendocrine cell in vitro. However, the method described in Patent Document 1 is a method of inducing differentiation of an enteroendocrine cell from a mammalian postnatal cell population including somatic stem cells such as intestinal stem cells, and in the first place, the frequency of the presence of intestinal stem cells and somatic stem cells equivalent thereto in vivo is very low. In addition, it is difficult to sort intestinal stem cells from a living body, particularly in a human.

An object to be achieved by the present invention is to provide a method of producing an enteroendocrine cell by using an intestinal stem cell which has been induced from a pluripotent stem cell. Further, an object to be achieved by the present invention is to provide a method of analyzing a disease mechanism, an evaluation method for a test substance, and a screening method for a therapeutic drug for a digestive system disease, which use the enteroendocrine cells produced by the method according to the present invention. Further, an object to be achieved by the present invention is to provide an enteroendocrine cell derived from a pluripotent stem cell, a culture medium or a culture medium kit, and a kit for producing an enteroendocrine cell.

As a result of diligent studies to achieve the above objects, the inventors of the present invention found that an enteroendocrine cell can be produced by culturing an intestinal stem cell or an intestinal epithelial cell, which is induced from a pluripotent stem cell, by using a culture medium that substantially does not contain a ligand of an epidermal growth factor but contains a cAMP activator and a DNA methyltransferase inhibitor. The present invention has been completed based on the above findings.

That is, according to the present invention, the following inventions are provided.
<1> A method of producing an enteroendocrine cell, the method comprising:
   culturing an intestinal stem cell or an intestinal epithelial cell, which is induced from a pluripotent stem cell, by using a culture medium that substantially does not contain a ligand of an epidermal growth factor but contains a cAMP activator and a DNA methyltransferase inhibitor.
<2> The method according to <1>, in which the cAMP activator is forskolin, and the DNA methyltransferase inhibitor is 5-aza-2'-deoxycytidine.
<3> The method according to <1> or <2>, in which the culture medium contains any one or more of a transforming growth factor-β signal inhibitor and a MEK inhibitor.
<4> The method according to <3>, in which the transforming growth factor-β signal inhibitor is A83-01, and the MEK inhibitor is PD98059 and/or PD0325901.
<5> The method according to any one of <1> to <4>, in which the culture medium contains a Notch inhibitor.
<6> The method according to <5>, in which the Notch inhibitor is LY411575.
<7> The method according to any one of <1> to <6>, in which the culture medium does not contain any one or more of a Wnt activator and a Wnt inhibitor.
<8> The method according to any one of <1> to <7>, in which the culture medium does not contain any one or more of a retinoic acid signal pathway activator and a Sonic hedgehog signal pathway inhibitor.
<9> The method according to any one of <1> to <8>, in which the pluripotent stem cell is a human induced pluripotent stem cell.
<10> The method according to any one of <1> to <9>, in which the culture step using the culture medium does not include a suspension culture step.
<11> The method according to any one of <1> to <10>, in which spheroids are not formed in the culture step using the culture medium.
<12> The method according to any one of <1> to <11>, in which the enteroendocrine cell to be produced has a sheet shape.
<13> A method of analyzing a disease mechanism, the method comprising:
   a step of producing an enteroendocrine cell by the method according to any one of <1> to <12>; and
   a step of analyzing a disease mechanism by using the enteroendocrine cell.
<14> An evaluation method for a test substance, comprising:
   a step of producing an enteroendocrine cell by the method according to any one of <1> to <12>; and
   a step of quantifying a hormone that is secreted by the enteroendocrine cell in a presence of a test substance.
<15> A screening method for a therapeutic drug for a digestive system disease, the screening method comprising:
   a step of producing an enteroendocrine cell by the method according to any one of <1> to <12>; and
   a step of culturing the enteroendocrine cell in a presence of a digestive system disease.
<16> An enteroendocrine cell derived from a pluripotent stem cell,
   in which the enteroendocrine cell expresses TPH1, PYY, CHGA, REG4, and NEUROG3 but does not express NKX6.1.
<17> A culture medium or a culture medium kit, comprising:
   a basal medium;
   serum or a serum substitute;
   a cAMP activator;
   a DNA methyltransferase inhibitor;
   a transforming growth factor-β signal inhibitor; and
   a MEK inhibitor,
   wherein the culture medium or the culture medium kit substantially does not contain a ligand of an epidermal growth factor.
<18> The culture medium or the culture medium kit according to <17>, further comprising:
   a Notch inhibitor.
<19> The culture medium or the culture medium kit according to <17> or <18>, in which the culture medium or the culture medium kit is for producing an enteroendocrine cell.
<20> A kit for producing an enteroendocrine cell, the kit comprising:
   the culture medium or the culture medium kit according to any one of <17> to <19>; and
   an intestinal stem cell or an intestinal epithelial cell, which is induced from a pluripotent stem cell.

According to the present invention, it is possible to produce an enteroendocrine cell by using, as a starting material, a pluripotent stem cell that has uniform properties and is capable of being produced in a large scale. In addition, it is possible to evaluate a gastrointestinal hormone producing ability of a test substance or the like by using the enteroendocrine cell produced by the method according to the aspect of the present invention. The culture medium or the culture medium kit, and the kit for producing an enteroendocrine cell according to the present invention can be used as a drug discovery support tool.

### BRIEF DESCRIPTION OF THE DRAWINGS

Fig. 1 shows gene expression of EEC markers in F-hiSIEC cells cultured in various culture media shown in Table 1.
Fig. 2 shows gene expression of EEC markers in F-hiSIEC cells cultured under the condition that 0.5 to 2.0 µmol/L of LY411575 or 5 to 10 µmol/L of DAPT has been added to the culture media of Table 2.
Fig. 3 shows gene expression of NKX6.1 in Caco-2 cells, cells (F-hiSIEC) obtained by culturing F-hiSIEC in a F-hiSIEC culture medium, and cells (EEC) obtained by culturing F-hiSIEC in a culture medium of Table 3 to induce it to EEC.

### DESCRIPTION OF THE PREFERRED EMBODIMENTS

Hereinafter, embodiments of the present invention will be described below.

The terms in the present specification have the following meanings.
5-aza-2'dc: 5-aza-2'-deoxycytidine
bFGF: Basic Fibroblast Growth Factor
BMP4: Bone morphogenetic protein 4
cAMP: Cyclic adenosine monophosphate
CHGA: Chromogranin A
Cript: Cysteine-rich PDZ-binding protein
DMEM: Dulbecco's Modified Eagle's Medium
DNMT: DNA methyltransferase
EGF: Epidermal Growth Factor
FBS: Fetal Bovine Serum
Fbxo15: F-Box Protein 15
FGF2: Fibroblast Growth Factor-2
GLP: Glucagon-like peptide
GSK-3β: Glycogen synthase kinase 3β
LIF: Leukemia inhibitory factor
MEK1: MAPK-ERK kinase 1
NEAA: Non-Essential Amino Acids
NEUROG3: Neurogenin 3
NKX6.1: Homeobox protein Nkx-6.1
PKA: Protein kinase A
PYY: Peptide YY
REG4: Regenerating islet-derived protein 4
ROCK: Rho-associated protein kinase
SCF: Stem cell factor
TGFβ: Transforming Growth Factor-β
TPH1: Tryptophan hydroxylase 1
VEGF: Vascular endothelial growth factor
Wnt: Wingless-type MMTV integration site family

### <Method of producing enteroendocrine cell>

A method of producing an enteroendocrine cell according to the present invention is a method including culturing an intestinal stem cell or an intestinal epithelial cell, which is induced from a pluripotent stem cell, by using a culture medium that substantially does not contain a ligand of an epidermal growth factor but contains a cAMP activator and a DNA methyltransferase inhibitor.

In the present invention, it is possible to efficiently induce an enteroendocrine cell by using a culture medium that substantially does not contain a ligand of an epidermal growth factor but contains a cAMP activator and a DNA methyltransferase inhibitor.

In the present invention, an intestinal stem cell or an intestinal epithelial cell, which has been induced from a pluripotent stem cell, is used.

"Pluripotent stem cells" refer to cells having the ability (differentiation pluripotency) to differentiate into all cells that constitute a living body and the ability (the self-replication ability) to generate daughter cells having the same differentiation potency as that of the pluripotent stem cells through cell division. Differentiation pluripotency can be evaluated by transplanting cells to be evaluated into nude mice and testing for the presence or absence of formation of teratoma including cells of each of the three germ layers (ectoderm, mesoderm, and endoderm).

Examples of the pluripotent stem cell include an embryonic stem cell (an ES cell), an embryonic germ cell (an EG cell), and an induced pluripotent stem cell (an iPS cell); however, examples thereof are not limited thereto as long as a cell has both differentiation pluripotency and self-replication ability. An ES cell or an iPS cell is preferably used. An iPS cell is more preferably used. The pluripotent stem cell is preferably a mammalian (for example, a primate such as a human or a chimpanzee or a rodent such as a mouse or a rat) cell and particularly preferably a human cell. Accordingly, in the most preferred embodiment of the present invention, a human induced pluripotent stem cell (human iPS cell) is used as the pluripotent stem cell.

The ES cell can be established, for example, by culturing an early embryo before implantation, an inner cell mass constituting the early embryo, a single blastomere, or the like (Manipulating the Mouse Embryo A Laboratory Manual, Second Edition, Cold Spring Harbor Laboratory Press (1994); Thomson, J. A. et. al., Science, 282, 1145-1147 (1998)). As the early embryo, an early embryo produced by nuclear transplantation of a nucleus of a somatic cell may be used (Wilmut et al. (Nature, 385, 810 (1997)), Cibelli et al. (Science, 280, 1256 (1998)), Iriya et al (Protein Nucleic Acid Enzyme, 44, 892 (1999)), Baguisi et al. (Nature Biotechnology, 17, 456 (1999)), Wakayama et al. (Nature, 394, 369 (1998); Nature Genetics, 22, 127 (1999); Proc. Natl. Acad. Sci. USA, 96, 14984 (1999)), Rideout III et al. (Nature Genetics, 24, 109 (2000)), Tachibana et al. (Human Embryonic Stem Cells Derived by Somatic Cell Nuclear Transfer, Cell (2013) in press). As the early embryo, a parthenogenetic embryo may be used (Kim et al. (Science, 315, 482-486 (2007)), Nakajima et al. (Stem Cells, 25, 983-985 (2007)), Kim et al. (Cell Stem Cell, 1, 346-352 (2007)), Revazova et al. (Cloning Stem Cells, 9, 432-449 (2007)), Revazova et al. (Cloning Stem Cells, 10, 11-24 (2008)). In addition to the above-described papers, the production of the ES cell is described in Strelchenko N., et al. Reprod Biomed Online. 9: 623-629, 2004; Klimanskaya I., et al. Nature 444: 481-485, 2006; Chung Y. et al., Cell Stem Cell 2: 113-117, 2008; Zhang X., et al. Stem Cells 24:2669-2676, 2006; Wassarman, P. M. et al. Methods in Enzymology, Vol. 365, 2003, or the like. In addition, fused ES cells obtained by cell fusion of ES cells with somatic cells are also included in the embryonic stem cells used in the method according to the embodiment of the present invention.

Some ES cells are available from conservation institutions or are commercially available. For example, human ES cells are available from the Institute for Frontier Medical Sciences, Kyoto University (for example, KhES-1, KhES-2, and KhES-3), WiCell Research Institute, ESI BIO.

An EG cell can be established by culturing a primordial germ cell in the presence of LIF, bFGF, and SCF (Matsui et al., Cell, 70, 841-847 (1992), Shamblott et al., Proc. Natl. Acad. Sci. USA, 9523, 13726-13731 (1998), Turnpenny et al., Stem Cells, 21 (5), 598-609, (2003)).

"Induced pluripotent stem cells (iPS cells)" are cells that have pluripotency (multiple differentiation potency) and proliferation ability and that are produced by reprogramming somatic cells by introducing reprogramming factors or the like. The induced pluripotent stem cells exhibit properties similar to the ES cells. The somatic cells used for producing iPS cells are not particularly limited and may be differentiated somatic cells or undifferentiated stem cells. In addition, the origin of the somatic cells is not particularly limited but preferably somatic cells of mammals (for example, primates such as a human and a chimpanzee, rodents such as a mouse and a rat) and particularly preferably human somatic cells. For example, a human adult-derived somatic cell (that is, a mature somatic cell) may be used in addition to the fetal somatic cell. Examples of the somatic cell include: (1) tissue stem cells (somatic stem cells) such as a neural stem cell, a hematopoietic stem cell, a mesenchymal stem cell, and a dental pulp stem cell; (2) tissue progenitor cells; and (3) differentiated cells such as a fibroblast (a skin cell and the like), an epithelial cell, a hepatocyte, a lymphocyte (T cell or B cell), an endothelial cell, a muscle cell, a hair cell, a gastric mucosal cell, an intestinal cell, a splenocyte, a pancreatic cell (an exocrine pancreatic cell and the like), a brain cell, a lung cell, a kidney cell, and a skin cell. The iPS cells can be produced by various methods reported so far. In addition, it is naturally expected that an iPS cell production method to be developed in the future will be applied.

The most basic producing method for an iPS cell is a method in which four transcription factors, Oct3/4, Sox2, Klf4, and c-Myc are introduced into a cell using a virus (Takahashi K, Yamanaka S: Cell 126 (4), 663-676, 2006; Takahashi, K, et al.: Cell 131 (5), 861-72, 2007). It has been reported that human iPS cells have been established by introducing four factors, Oct4, Sox2, Lin28, and Nanog (Yu J, et al.: Science 318 (5858), 1917-1920, 2007). It has also been reported that iPS cells have been established by introducing three factors excluding c-Myc (Nakagawa M, et al.: Nat. Biotechnol. 26 (1), 101-106, 2008), two factors of Oct3/4 and Klf4 (Kim J B, et al.: Nature 454 (7204), 646-650, 2008), or Oct3/4 alone (Kim J B, et al.: Cell 136 (3), 411-419, 2009). In addition, a method for introducing a protein, which is an expression product of a gene, into cells (Zhou H, Wu S, Joo J Y, et al.: Cell Stem Cell 4, 381-384, 2009; Kim D, Kim C H, Moon J I, et al.: Cell Stem Cell 4, 472-476, 2009) has also been reported. On the other hand, it has also been reported that by using BIX-01294 which is an inhibitor of histone methyltransferase G9a, valproic acid (VPA) which is a histone deacetylase inhibitor, or Bay K8644, the production efficiency has been improved and the factors to be introduced have been reduced (Huangfu D, et al.: Nat. Biotechnol. 26 (7), 795-797, 2008; Huangfu D, et al.: Nat. Biotechnol. 26 (11), 1269-1275, 2008; Silva J, et al.: PLoS. Biol. 6 (10), e253, 2008). Studies on gene transfer methods have also been carried out, and technologies for gene transfer have been developed using, in addition to a retrovirus, a lentivirus (Yu J, et al.: Science 318 (5858), 1917-1920, 2007), an adenovirus (Stadtfeld M, et al.: Science 322 (5903), 945-949, 2008), a plasmid (Okita K, et al.: Science 322 (5903), 949-953, 2008), a transposon vector (Woltjen K, Michael I P, Mohseni P, et al.: Nature 458, 766-770, 2009; Kaji K, Norrby K, Paca A, et al.: Nature 458, et al. 771-775, 2009; Yusa K, Rad R, Takeda J, et al.: Nat Methods 6, 363-369, 2009) or an episomal vector (Yu J, Hu K, Smuga-Otto K, Tian S, et al.: Science 324, 797-801, 2009).

Cells transformed to iPS cells, that is, cells that have undergone initialization (reprogramming) can be selected using, as an indicator, the expression of pluripotent stem cell markers (undifferentiation markers) such as Fbxo15, Nanog, Oct3/4, Fgf-4, Esg-1, and Cript, or the like. The selected cells are recovered as iPS cells.

The iPS cells can be available from, for example, National University Corporation Kyoto University, or Independent Administrative Institution RIKEN BioResource Center.

In the present specification, "inducing differentiation" refers to acting to differentiate along a specific cell lineage. In the present invention, an intestinal stem cell or an intestinal epithelial cell, which has been induced to be differentiated from a pluripotent stem cell, is used.

### <<Differentiation from pluripotent stem cell into intestinal stem cell>>

An intestinal stem cell can be produced by culturing a pluripotent stem cell under conditions in which the pluripotent stem cell is induced to be differentiated into an intestinal stem cell. The culture conditions are not particularly limited as long as the pluripotent stem cells differentiate into intestinal stem cells. Typically, differentiation induction of two stages described below, that is, the differentiation of the pluripotent stem cells into endoderm-like cells (a step (1-1)) and the differentiation of endoderm-like cells into intestinal stem cells (a step (1-2)) are carried out so that the pluripotent stem cells differentiate into intestinal stem cells via endoderm-like cells.

### Step (1-1) differentiation into endoderm-like cell

In this step, pluripotent stem cells are cultured and differentiated into endoderm-like cells. In other words, the pluripotent stem cells are cultured under conditions that induce differentiation into the endoderm-like cells. The culture conditions are not particularly limited as long as the pluripotent stem cells differentiate into endoderm-like cells. For example, the pluripotent stem cells are cultured in a culture medium to which activin A is added, according to a conventional method. In this case, the concentration of activin A in the culture medium is set to, for example, 10 ng/mL to 200 ng/mL and preferably 20 ng/mL to 150 ng/mL. It is preferable to add serum or a serum substitute (KnockOut^{™} Serum Replacement (KSR) or the like) to the culture medium from the viewpoints of the cell growth rate and cell maintenance. The serum is not limited to fetal bovine serum, and human serum, sheep serum, or the like can be also used. The addition amount of serum or a serum substitute is, for example, 0.1% (v/v) to 10% (v/v).

An inhibitor of the Wnt/β-catenin signal pathway (for example, hexachlorophene, quercetin, or Wnt3a which is a Wnt ligand) may be added to the culture medium to promote differentiation into endoderm-like cells.

One or more of BMP4, VEGF, and FGF2 may be added to the culture medium to promote differentiation into endoderm-like cells.

This step can be also carried out by the method described in WO2014/165663A or a method based thereon.

In a preferred aspect, two stage culture is carried out as the step (1-1). First stage culture is carried out in a culture medium to which a relatively low concentration of serum (for example, 0.1% (v/v) to 1% (v/v)) is added, and subsequent second stage culture is carried out in a culture medium having a higher serum concentration than the first stage culturing (for example, a serum concentration of 1% (v/v) to 10% (v/v)). Employing the two stage culture in this manner is preferable since the proliferation of undifferentiated cells is suppressed by the first stage culture and differentiated cells are proliferated by the subsequent second stage culture.

The period (the culture period) of the step (1-1) is, for example, 1 day to 10 days and preferably 2 days to 7 days. In a case where the two stage culture is employed as the step (1-1), the culture period of first stage is, for example, 1 day to 7 days and preferably 2 days to 5 days, and the culture period of second stage is, for example, 1 day to 6 days and preferably 1 day to 4 days.

### Step 1-2: differentiation into intestinal stem cell

In this step, the endoderm-like cells obtained in the step (1-1) are cultured and differentiated into intestinal stem cells. In other words, the endoderm-like cells are cultured under conditions that induce differentiation into intestinal stem cells. The culture conditions are not particularly limited as long as the endoderm-like cells differentiate into intestinal stem cells. The culture is preferably carried out in the presence of FGF2 (fibroblast growth factor 2) or in the presence of a GSK-3β inhibitor. Human FGF2 (for example, a human recombinant FGF2) is preferably used as FGF2.

Typically, the cell population or a part thereof obtained through the step (1-1) is used in the step (1-2) without sorting. Alternatively, the step (1-2) may be carried out after sorting endoderm-like cells from the cell population obtained through step (1-1). The sorting of endoderm-like cells may be carried out, for example, with a flow cytometer (cell sorter) using a cell surface marker as an indicator.

"In the presence of FGF2" is synonymous with under the condition in which FGF2 is added to a culture medium. Accordingly, in order to carry out culture in the presence of FGF2, a culture medium to which FGF2 is added may be used. An example of the concentration of FGF2 to be added is 100 ng/mL to 500 ng/mL.

Similarly, "in the presence of a GSK-3β inhibitor" is synonymous with under the conditions in which a GSK-3β inhibitor has been added to a culture medium. Accordingly, in order to carry out culture in the presence of GSK-3β inhibitor, a culture medium to which a GSK-3β inhibitor has been added may be used. Examples of the GSK-3β inhibitor include CHIR99021, SB216763, CHIR98014, TWS119, Tideglusib, SB415286, BIO, AZD2858, AZD1080, AR-A014418, TDZD-8, LY2090314, IM-12, Indirubin, Bikinin, and 1-Azakenpaullone. The concentration of the GSK-3β inhibitor to be added may be 0.1 nmol/L to 30 µmol/L, and it is preferably 1 nmol/L to 10 µmol/L, more preferably 10 nmol/L to 10 µmol/L, and still more preferably 50 nmol/L to 5 µmol/L.

The period (the culture period) of the step (1-2) is, for example, 2 days to 10 days and preferably 3 days to 7 days. In a case where the culture period is too short, an expected effect (increase in differentiation efficiency or promotion of acquisition of functions as intestinal stem cells) cannot be sufficiently obtained. On the other hand, in a case where the culture period is too long, the differentiation efficiency will be reduced.

The differentiation into intestinal stem cells can be determined or evaluated using, for example, the expression of an intestinal stem cell marker as an indicator. Examples of the intestinal stem cell marker include G protein-coupled receptor 5 (LGR5) containing leucine-rich repeats and Ephrin B2 receptor (EphB2).

The cell density at the end of the first differentiation step may be 5.0 × 10⁴ cells/cm² to 20 × 10⁴ cells/cm², and it is preferably 6.0 × 10⁴ cells/cm² to 16 × 10⁴ cells/cm² and more preferably 7.0 × 10⁴ cells/cm² to 14 × 10⁴ cells/cm².

The intestinal stem cells obtained in the step (1-2) can also be cryopreserved. The cryopreserved intestinal stem cells may be thawed and a subsequent step (the proliferation step or the like) may be carried out. For the cryopreservation of the cells, a solution generally used for the cryopreservation of cultured cells can be used, and a culture medium, DMSO, serum, a general cryopreservation reagent, and the like may be contained. Examples of the general cryopreservation reagent include CryoStor (registered trade name) Freeze Media, CellBanker (registered trade name), FreSR (trademark)-S, NutriFreez (trademark) D10 Cryopreservation Medium, Cellvation, Cryoscarless (registered trade name), and Bambanker (registered trade name).

### <<Differentiation from intestinal stem cell into intestinal epithelial cell>>

The differentiation step of intestinal epithelial cells from the intestinal stem cells preferably includes a step of differentiating the intestinal stem cells into the intestinal epithelial cell in the presence of a MEK1 inhibitor, a DNA methyltransferase inhibitor, a TGFβ receptor inhibitor, EGF, and a cAMP activator.

It is possible to positively increase the intracellular cAMP level by using a cAMP activator. "In the presence of a MEK1 inhibitor, a DNA methyltransferase inhibitor, a TGFβ receptor inhibitor, EGF, and a cAMP activator" refers to that all these compounds are required in order to carry out one or more cultures constituting the second differentiation step, and it is not required, as an essential condition, that all of these compounds are used at the same time, that is, that the culture using a culture medium to which all of these compounds are added is carried out.

The differentiation into intestinal epithelial cells can be constituted by one or two or more cultures. In each culture constituting the differentiation step into intestinal epithelial cells, it is possible to use, for example:
a culture medium to which EGF has been added as an essential component;
a culture medium to which EGF and a ROCK inhibitor (for example, Y-27632) have been added as essential components;
a culture medium to which EGF and a cAMP activator (for example, forskolin) has been added as essential components;
a culture medium to which EGF, a MEK1 inhibitor, a DNA methyltransferase inhibitor, and a TGFβ receptor inhibitor are added as essential components; and
a culture medium to which EGF, a cAMP activator (for example, forskolin), a MEK1 inhibitor, a DNA methyltransferase inhibitor, and a TGFβ receptor inhibitor have been added as essential components.

Examples of the MEK1 inhibitor include PD98059, PD184352, PD184161, PD0325901, U0126, MEK inhibitor I, MEK inhibitor II, MEK1/2 inhibitor II, and SL327. Examples of the DNA methyltransferase inhibitor include 5-aza-2'-deoxycytidine, 5-azacytidine, RG108, and zebularine. Regarding the TGFβ receptor inhibitor, considering that A8301 used in Examples described later exhibits an inhibitory activity on TGF-β receptors ALK4, ALK5, and ALK7, it is preferable to use an inhibitor that exhibits an inhibitory activity on one or more of TGF-β receptors, ALK4, ALK5, and ALK7. For example, A8301, SB431542, SB-505124, SB525334, D4476, ALK5 inhibitor, LY2157299, LY364947, GW788388, and RepSox satisfy the above conditions. As the cAMP activator, forskolin, indomethacin, NKH477 (colforsin daropate), a cell-derived toxin protein (pertussis toxin, cholera toxin), PACAP-27, PACAP-38, SKF83822, or the like can be used. Forskolin, NKH477 (colforsin daropate), cholera toxin, PACAP-27, PACAP-38, and SKF83822 exhibit an adenylate cyclase activating activity and promote the intracellular cAMP synthesis.

An example of the concentration of the MEK1 inhibitor to be added (in a case of PD98059) is 4 µmol/L to 100 µmol/L and preferably 10 µmol/L to 40 µmol/L. An example of the concentration of the DNA methyltransferase inhibitor (in a case of 5-aza-2'-deoxycytidine) to be added is 1 µmol/L to 25 µmol/L and preferably 2.5 µmol/L to 10 µmol/L, and an example of the concentration of the TGFβ receptor inhibitor (in a case of A8301) to be added is 0.1 µmol/L to 2.5 µmol/L and preferably 0.2 µmol/L to 1 µmol/L. An example of the concentration of EGF to be added is 5 ng/mL to 100 ng/mL and preferably 10 ng/mL to 50 ng/mL. In addition, an example of the concentration of the cAMP activator to be added (in a case of forskolin) is 1 µmol/L to 200 µmol/L and preferably 5 µmol/L to 100 µmol/L. In addition, the concentration of compounds in a case of using compounds different from the exemplified compounds, that is, PD98059, 5-aza-2'-deoxycytidine, A8301, and forskolin, can be set according to the above-described concentration range by those skilled in the art in consideration of the difference in the properties (especially the difference in activity) between the compound to be used and the exemplified compounds (PD98059, 5-aza-2'-deoxycytidine, A8301, and forskolin).

The step of differentiation into intestinal epithelial cells may be carried out, in addition to the conditions described above, under the condition in which cAMP is supplied to cells (referred to as an "additional condition 1") and the condition in which a cAMP-degrading enzyme inhibitor is present (referred to as an "additional condition 2"), or under any of these conditions. The additional condition 1 (the condition in which cAMP is supplied to cells) is synonymous with the condition in which a compound capable of being incorporated into cells and acting as cAMP when taken up into cells is present. Accordingly, in order to satisfy the additional condition 1, for example, a culture medium to which a cAMP derivative that can be incorporated into cells is added may be used. In a case where the additional condition 1 is adopted, it can be expected that the decrease in the intracellular cAMP concentration is suppressed, and thus the induction of differentiation into intestinal epithelium cell, in particular, the acquisition of a function as intestinal epithelial cells is promoted. That is, the above condition may enable the preparation of more functional intestinal epithelial cells. As a cAMP derivative, it is possible to employ a PKA activator (for example, 8-Br-cAMP (a 8-bromoadenosine-3',5'-cyclic monophosphate sodium salt, CAS Number: 76939-46-3), 6-Bnz-cAMP (an N6-benzoyladenosine-3',5'-cyclic monophosphate sodium salt, CAS Number: 1135306-29-4), cAMPS-Rp (an (R)-adenosine cyclic 3',5'-(hydrogen phosphorothioate)triethlylamminium salt, CAS Numer: 151837-09-1), cAMPS-Sp (an (S)-adenosine cyclic 3',5'-(hydrogen phosphorothioate)triethylammanium salt, CAS Number: 93602-66-5), dibutyryl-cAMP (an N6,O2'-dibutyryladenosine 3',5'-cyclic monophosphate solidium salt, CAS Number: 16980-89-5), 8-Cl-cAMP (a 8-chloroadenosine-3',5'-cyclic monophosphate salt, CAS Number: 124705-03-9)), an Epac activator (Rp-8-Br-cAMPS (8-bromodenosine3',5',-cyclic monophosphothioate, an Rp-isomer sodium salt, CAS Number: 129735-00-8), 8-CPT-cAMP (8-(4-chlorophenylthio)adenosine 3',5'-cyclic monophosphate, CAS Number: 93882-12-3), or 8-pCPT-2'-O-Me-cAMP (8-(4-chlorophenylthio)-2'-O-methyladenosine 3',5'-cyclic monophosphate monosodium, CAS Number: 634207-53-7), or the like). An example of the concentration of the cAMP derivative (in a case of 8-Br-cAMP) to be added is 0.1 mmol/L to 10 mmol/L, and it is preferably 0.2 mmol/L to 5 mmol/L and more preferably 0.5 mmol/L to 2 mmol/L. In addition, in a case of using a compound different from the exemplified compound, that is, 8-Br-cAMP, the addition concentration can be set according to the above-described concentration range by those skilled in the art in consideration of the difference in the properties (especially the difference in activity) between the compound to be used and the exemplified compound (8-Br-cAMP).

The additional condition 2 (the condition in which a cAMP-degrading enzyme inhibitor is present) is synonymous with the condition in which the cAMP-degrading enzyme inhibitor is added to the culture medium. In a case where the additional condition 2 is adopted, it can be expected that the decrease in the intracellular cAMP concentration is suppressed by the inhibition of cAMP degradation and thus the induction of differentiation into intestinal epithelium cell, in particular, the acquisition of a function as intestinal epithelial cells is promoted. That is, the above condition may enable the preparation of more functional intestinal epithelial cells. In addition, in a case where the additional conditions 1 and 2 are used in combination, it is possible to suppress the decrease in intracellular cAMP concentration while supplying cAMP to cells. As a result, the condition becomes effective for maintaining intracellular cAMP at a high level, and it can be expected that efficient induction of differentiation into intestinal epithelial cells is promoted.

Examples of the cAMP-degrading enzyme inhibitor include IBMX (3-isobutyl-1-methylxanthine) (MIX), Theophylline, Papaverine, Pentoxifilline (Trental), KS-505, 8-methoxymethyl-IBMX, Vinpocetine (TCV-3B), EHNA, Trequinsin (HL-725), Lixazinone (RS-82856), (LY-186126), Cilostamide (OPC3689), Bemoradan (RWJ-22867), Anergrelide (BL4162A), Indolidan (LY195115), Cilostazol (OPC-13013), Milrinone (WIN47203), Siguazodan (SKF-94836), 5-methyl-imazodan (CI 930), SKF-95654, Pirilobendan (UD-CG 115 BS), Enoximone (MDL 17043), Imazodan (CL 914), SKF-94120, Vesnarinone (OPC 8212), Rolipram (Ro-20-1724), (ZK-62711), Denbufyll'ine, Zaprinast (M&B-22,948), Dipyridamole, Zardaverine, and AH-21-132, Sulmazol (AR-L 115 BS). An example of the concentration of the cAMP-degrading enzyme inhibitor (in a case of IBMX) to be added is 0.05 mmol/L to 5 mmol/L, and it is preferably 0.1 mmol/L to 3 mmol/L and more preferably 0.2 mmol/L to 1 mmol/L.

The period (culture period) of the step of differentiation into intestinal epithelial cells is, for example, 7 days to 40 days and preferably 7 days to 30 days. In a case where the culture period is too short, an expected effect (increase in differentiation efficiency or promotion of acquisition of functions as intestinal epithelial cells) cannot be sufficiently obtained. On the other hand, in a case where the culture period is too long, the differentiation efficiency will be reduced.

The differentiation into intestinal epithelial cells can be determined or evaluated using, for example, the expression of an intestinal epithelial cell marker, the incorporation of a peptide, or the induction of the expression of a drug metabolizing enzyme via a vitamin D receptor as an indicator. Examples of the intestinal epithelial cell marker include ATP-binding cassette transporter B1/Multidrug resistance protein 1 (ABCB1/MDR1), ATP-binding cassette transporter G2/Breast cancer resistance protein (ABCG2/BCRP), cytochrome P4503A4 (CYP3A4), Fatty acid binding protein 2 (FABP2), Pregnane X receptor (PXR), Solute carrier (SLC) family member 5A1/Sodium-coupled glucose transporter 1 (SLC5A1/SGLT1), Solute carrier (SLC) family member 15A1/Peptide transporter 1 (SLC15A1/PEPT1), Solute carrier (SLC) organic anion transporter 2B1 (SLCO₂B1/OATP2B1), Sucrase-isomaltase, Uridine diphosphate glucuronosyltransferase 1A1 (UGT1A1), Uridine diphosphate glucuronosyltransferase 1A4 (UGT1A4), Villin 1, and Carboxylesterase 2A1 (CES2A1). Among these, Sucrase-isomaltase and Villin 1 which are highly specific to the intestinal epithelium cell, CYP3A4 which is a major drug metabolizing enzyme in the small intestine, SLC15A1/PEPT1 which is involved in peptide absorbance in the small intestine, SLC5A1/SGLT1, a glucose transporter, which is expressed at the apical membrane side of the small intestine, SLCO2B1/OATP2B1 which is involved in the absorbance of organic anions in the small intestine, and CES2A1, a hydrolase, which is highly expressed in the small intestine, are particularly effective markers.

To obtain a cell population consisting only of target cells (intestinal epithelial cells) or a cell population including the target cells in a high proportion (high purity), a cell population after culture may be selected and sorted using a cell surface marker characteristic of the target cells as an indicator.

In the present invention, the intestinal stem cell or the intestinal epithelial cell, which is induced from a pluripotent stem cell as described above, is cultured by using a culture medium that substantially does not contain a ligand of an epidermal growth factor but contains a cAMP activator and a DNA methyltransferase inhibitor.

Examples of the ligand of the epidermal growth factor (EGFR) include EGF, betacellulin, amphiregulin, TNF-α, and HB-EGF.

As the cAMP activator, forskolin, indomethacin, NKH477 (colforsin daropate), a cell-derived toxin protein (pertussis toxin, cholera toxin), PACAP-27, PACAP-38, SKF83822, or the like can be used. Forskolin, NKH477 (colforsin daropate), cholera toxin, PACAP-27, PACAP-38, and SKF83822 exhibit an adenylate cyclase activating activity and promote the intracellular cAMP synthesis.

An example of the concentration of the cAMP activator to be added (in a case of forskolin) is 1 µmol/L to 200 µmol/L and preferably 5 µmol/L to 100 µmol/L. In a case of using a compound different from forskolin, the addition concentration can be set according to the above-described concentration range by those skilled in the art in consideration of the difference in the properties (especially the difference in activity) between the compound to be used and forskolin.

As the DNA methyltransferase inhibitor, 5-aza-2'-deoxycytidine, 5-azacytidine, RG108, zebularine, or the like can be used.

An example of the concentration of the DNA methyltransferase inhibitor to be added (in a case of 5-aza-2'-deoxycytidine) is 0.1 µmol/L to 50 µmol/L and preferably 0.5 µmol/L to 20 µmol/L. In a case of using a compound different from 5-aza-2'-deoxycytidine, the addition concentration can be set according to the above-described concentration range by those skilled in the art in consideration of the difference in the properties (especially the difference in activity) between the compound to be used and 5-aza-2'-deoxycytidine.

The culture medium that substantially does not contain a ligand of an epidermal growth factor but contains a cAMP activator and a DNA methyltransferase inhibitor may contain any one or more of a transforming growth factor-β signal inhibitor and a MEK inhibitor.

As the transforming growth factor-β signal inhibitor, it is possible to use, for example, A83-01, SB431542, SB-505124, SB525334, D4476, ALK5 inhibitor, LY2157299, LY364947, GW788388, or RepSox.

The concentration of the transforming growth factor-β signal inhibitor (in a case of A83-01) to be added may be 0.1 nmol/L to 30 µmol/L, and it is preferably 1 nmol/L to 10 µmol/L, more preferably 10 nmol/L to 10 µmol/L, and still more preferably 50 nmol/L to 5 µmol/L. In a case of using a compound different from A83-01, the addition concentration can be set according to the above-described concentration range by those skilled in the art in consideration of the difference in the properties (especially the difference in activity) between the compound to be used and A83-01.

As the MEK inhibitor, PD98059, PD184352, PD184161, PD0325901, U0126, MEK inhibitor I, MEK inhibitor II, MEK1/2 inhibitor II, SL327, or the like can be used.

An example of the concentration of the MEK inhibitor to be added (in a case of PD98059) is 1 µmol/L to 200 µmol/L and preferably 5 µmol/L to 100 µmol/L. In a case of using a compound different from PD98059, the addition concentration can be set according to the above-described concentration range by those skilled in the art in consideration of the difference in the properties (especially the difference in activity) between the compound to be used and PD98059.

The culture medium that substantially does not contain a ligand of an epidermal growth factor but contains a cAMP activator and a DNA methyltransferase inhibitor may contain a Notch inhibitor.

As the Notch inhibitor, LY411575, DAPT, a γ-secretase inhibitor XXI (compound E) (CAS number: 209986-17-4), a γ-secretase inhibitor I (CAS number: 133407-83-7), dibenzazepine, or RO4929097 can be used. The Notch inhibitor is preferably LY411575.

An example of the concentration of the Notch inhibitor to be added (in a case of LY411575) is 0.1 µmol/L to 10 µmol/L and preferably 0.5 µmol/L to 5 µmol/L. In a case of using a compound different from LY411575, the addition concentration can be set according to the above-described concentration range by those skilled in the art in consideration of the difference in the properties (especially the difference in activity) between the compound to be used and LY411575.

It is preferable that the culture medium that substantially does not contain a ligand of an epidermal growth factor but contains a cAMP activator and a DNA methyltransferase inhibitor does not contain any one or more of a Wnt activator and a Wnt inhibitor.

Examples of the Wnt activator include CHIR99021, LY2090314, NP031112 (tideglusib), lithium, A1070722, SSKL2001, R-Spondin 1, Wnt3a, and a Gsk inhibitor.

Examples of the Wnt inhibitor include Wnt-C59, a Dkk family protein (for example, at least one of Dkk-1, Dkk2, Dkk3, or Dkk4), sFRP (for example, at least one of sFRP-1 or sFRP-2), an antibody against a Wnt receptor such as OMP-18R5, LGK974, CWP232291, PR-724, IQR-1, IWP2, IWP-L6, ICG-001, WIKI4, Ky02111, FH535, XAV939, NSC668036, FJ9, and 3289-8625.

The culture medium that substantially does not contain a ligand of an epidermal growth factor but contains a cAMP activator and a DNA methyltransferase inhibitor may contain a histone deacetylase inhibitor or may not contain it.

Examples of the histone deacetylation enzyme inhibitor include Tubastatin A, ACY1215, valproic acid, SAHA, trichostatin A, SHBA, CBHA, LAQ-824, PDX-101, LBH-589, ITF2357, PCI-24781, Compound 7 (ChemieTek, LLC), JNK-24681585 (quisinostat), SB939 (pracinostat), 4SC-201 (resminostat), tefinostat (CHR-2845), CHR-3996, CG200745, a depsipeptide (romidepsin), a butyrate, MS-275, MGCD0103, and CI994.

It is preferable that the culture medium that substantially does not contain a ligand of an epidermal growth factor but contains a cAMP activator and a DNA methyltransferase inhibitor does not contain any one or more of a retinoic acid signal pathway activator and a Sonic hedgehog signal pathway inhibitor.

Examples of the retinoic acid signal pathway activator include retinoic acid, a retinoic acid derivative, an all-trans-type retinoic acid, a synthetic retinoid ec23, Ch55, TTNPB, fenretinide, AC261066, adapalene, AC55649, AM80, AM580, BMS753, and tazarotene.

Examples of the Sonic hedgehog signal pathway inhibitor include SANT-1, SANT-2, JK-184, and jervine.

In the present invention, the period for carrying out culture using the culture medium that substantially does not contain a ligand of an epidermal growth factor but contains a cAMP activator and a DNA methyltransferase inhibitor is, for example, 2 days to 20 days and preferably 4 days to 15 days.

It is preferable that the culture step using the culture medium that substantially does not contain a ligand of an epidermal growth factor but contains a cAMP activator and a DNA methyltransferase inhibitor does not include the suspension culture step. In addition, it is preferable that cells do not form spheroids in the culture step using the culture medium that substantially does not contain a ligand of an epidermal growth factor but contains a cAMP activator and a DNA methyltransferase inhibitor.

It is noted that in a case of recovering cells in association with culture medium exchange or subculture, cells may be treated in advance with a ROCK (Rho-associated coiled-coil forming kinase) inhibitor such as Y-27632 to suppress cell death.

In the present invention, other culture conditions (such as the culture temperature) may be conditions generally employed in culturing animal cells. That is, culture may be carried out, for example, at 37°C in an environment of 5% CO₂.

In addition, as a basal medium, Iskov modified Dulbecco's culture medium (IMDM) (GIBCO-BRL or the like), Ham F12 culture medium (Ham F12) (SIGMA, Gibco-BRL, or the like), Dulbecco's modified Eagle's culture medium (DMEM) (Nacalai Tesque Inc., Sigma-Aldrich Co. LLC, Gibco-BRL or the like), Glasgow basal medium (Gibco-BRL or the like), RPMI1640 culture medium, or the like can be used. Two or more basal media may be used in combination.

Examples of the component that can be added to the culture medium include serum or a serum substitute, an antibiotic, 2-mercaptoethanol, PVA, non-essential amino acids (NEAA), insulin, transferrin, and selenium. Examples of the serum include bovine serum albumin (BSA) and fetal bovine serum (FBS). Examples of the serum substitute include Gibco (registered trade name) B-27 (registered trade name) Supplement.

Typically, cells are two-dimensionally cultured using a culture dish or the like. According to the method according to the embodiment of the present invention, enteroendocrine cells can be produced by two-dimensional culture (preferably, adhesion culture). The morphology of the enteroendocrine cells to be produced is not particularly limited; however, it is preferably a sheet shape.

In an example, the culture can be carried out in a culture container coated with an extracellular matrix of 3% by volume to 100% by volume. As the example of the extracellular matrix, it is possible to use Matrigel^{™}, fibronectin, laminin, vitronectin, tenascin, entactin, thrombospondin, elastin, gelatin, collagen, fibrin, merosin, ankyrin, chondronectin, link protein, bone sialoprotein, osteocalcin, osteopontin, epinectin, hyaluronectin, undulin, epiregulin, or kalinin can be used, where Matrigel^{™} is preferable used. Matrigel (registered trade name) is a product name of a gelatinous protein mixture that is secreted by Engelbreth-Holm-Swarm mouse sarcoma cells produced by Corning Life Sciences.

In a particularly preferred example of the present invention, intestinal epithelial cells (including intestinal stem cells, LGR5+ cells) derived from human iPS cells are seeded on a cell culture insert or a 96-well plate and, from the next day, cultured in a DMEM/F12 culture medium containing FBS, B27, HepExtend^{™}, forskolin, A83-01, 5-aza-2'-deoxycytidine, PD98059, and LY41157, whereby enteroendocrine cells can be produced.

### <enteroendocrine cell derived from pluripotent stem cell>

According to the present invention, there is provided an enteroendocrine cell derived from the pluripotent stem cell produced by the above-described method according to the embodiment of the present invention.

The differentiation into enteroendocrine cells can be determined or evaluated using, for example, the expression of an enteroendocrine cell marker as an indicator. Important examples of enteroendocrine cell markers are CHGA and REG4. In addition, the determination or the evaluation can be carried out by using, as an indicator, the expression of TPH1 or PYY, which is a factor involved in the hormone secretion which is a function of enteroendocrine cells, or NEUROG3, which is a factor involved in the development of endocrine cells in the intestine.

The enteroendocrine cell according to the embodiment of the present invention is characterized in that it expresses TPH1, PYY, CHGA, REG4, and NEUROG3 but does not express NKX6.1.

The presence or absence of expression of TPH1, PYY, CHGA, REG4, NEUROG3, and NKX6.1 can be measured by mRNA or protein level.

"Expressing TPH1, PYY, CHGA, REG4, and NEUROG3" means that mRNAs or proteins, which are expression products of TPH1, PYY, CHGA, REG4, and NEUROG3, can be detected by a known method.

"Not expressing NKX6.1" means that an mRNA or a protein, which is an expression product of NKX6.1, cannot be detected by a known method. However, this is not limited to a case where the expression level is 0 and includes a case where the expression level is very small. Examples of the case of "NKX6.1 is not expressed" include a case where the relative expression level with respect to the expression level of NKX6.1 in the human colon cancer-derived cell line CaCo-2 is 0.1 or less, preferably a case where it is 0.05 or less more, and more preferably a case where it is 0.01 or less more.

<Method of analyzing disease mechanism, evaluation method for test substance, and screening method for therapeutic drug for digestive system disease, which use enteroendocrine cell>

The present invention relates to a use application of the enteroendocrine cell produced by the method according to the embodiment of the present invention.

A first aspect includes a method of analyzing a disease mechanism, the method including a step of producing an enteroendocrine cell by the method according to the embodiment of the present invention; and a step of analyzing a disease mechanism by using the enteroendocrine cell.

A second aspect includes a screening method for a therapeutic drug for a digestive system disease, the screening method including a step of producing an enteroendocrine cell by the method according to the embodiment of the present invention, and a step of culturing the enteroendocrine cell in a presence of a digestive system disease.

A third aspect includes an evaluation method for a test substance, including a step of producing an enteroendocrine cell by the method according to the embodiment of the present invention, and a step of quantifying a hormone that is secreted by the enteroendocrine cell in a presence of a test substance.

The abnormal secretion of a gastrointestinal hormone produced from the endocrine cells is a factor of functional disorder (constipation, diagnosis, anorexia, and the like) of various organs of the whole body and various diseases (obesity, diabetes, and hypersensitivity enteritis). Therefore, the expression or production amount of an endocrine cell marker or a gastrointestinal hormone serves as a biomarker, and can be used as an indicator of various diseases. Examples the above-described evaluation method include a method of analyzing a disease mechanism by measuring the morphology, the number of cells, or the above-described biomarker in a case where a certain pathological model is created by addition of a drug to induced enteroendocrine cells or gene editing.

A therapeutic drug or a test substance can be screened or evaluated by bringing the therapeutic drug or the test substance into contact with enteroendocrine cells. The enteroendocrine cells produced by the method according to the embodiment of the present invention can be used for a model system of the intestinal tract, particularly the small intestine, and are useful for the evaluation of pharmacokinetics (absorption, metabolism, and the like) and toxicity in the intestinal tract, particularly the small intestine, and the evaluation of the response of the small intestine. That is, the enteroendocrine cells produced by the method according to the embodiment of the present invention can be used for the evaluation of in vivo kinetics of a therapeutic drug or a test substance, the evaluation of toxicity thereof, and the evaluation of response to a test substance.

Specifically, it is possible to test, by using enteroendocrine cells, the absorbability or membrane permeability of a therapeutic drug or a test substance, the drug interaction, the induction of a drug metabolizing enzyme, and the induction, toxicity, and immune response of a drug transporter, as well as the production of a hormone secreted by enteroendocrine cell, and the like. That is, the present invention provides, as one of the use applications of enteroendocrine cells, a method of evaluating the absorbability or membrane permeability of a therapeutic drug or a test substance, the drug interaction, the induction of a drug metabolizing enzyme, and the induction, toxicity, and immune response of a drug transporter, as well as the production of a hormone secreted by enteroendocrine cell, and the like. In the above method, the following steps are carried out: (i) a step of preparing enteroendocrine cells; (ii) a step of bringing a therapeutic drug or a test substance into contact with the enteroendocrine cells; and (iii) a step of quantifying the therapeutic drug or test substance that has permeated through the enteroendocrine cells, and evaluating the absorbability or membrane permeability of a therapeutic drug or a test substance, the drug interaction, the induction of a drug metabolizing enzyme, and the induction, toxicity, or immune response of a drug transporter or quantifying a production amount of a hormone secreted by enteroendocrine cell. Specific examples of the hormone that is secreted by the enteroendocrine cells include serotonin, glucagon-like peptide-1 (GLP-1), gastric inhibitory polypeptide (GIP), gastrin, and PYY. In addition, the absorbability of the therapeutic drug or the test substance can also be evaluated by the method described below.

In the step (i), enteroendocrine cells are typically cultured on a semipermeable membrane (porous membrane) to form a cell layer. Specifically, for example, by using a culture vessel equipped with a cell culture insert (for example, Transwell (registered trade name) provided by Corning Incorporated), cells are plated and cultured in the cell culture insert, and then a cell layer constituted of the enteroendocrine cells are obtained.

The "contact" in the step (ii) is typically carried out by adding a therapeutic drug or a test substance to a culture medium. The timing for adding the therapeutic drug or the test substance is not particularly limited. Accordingly, the therapeutic drug or the test substance may be added at a certain timing after starting culture in a culture medium containing no test substance, or the culture may be started in a culture medium containing the therapeutic drug or the test substance in advance.

As the therapeutic drug or the test substance, organic compounds or inorganic compounds having various molecular sizes can be used, and bacteria (enteric bacteria, lactic acid bacteria, and the like), viruses, and cells (immune cells, fibroblasts) can also be used. Examples of the organic compound include a nucleic acid, a peptide, a protein, a lipid (a simple lipid, a complex lipid, a phosphoglyceride, a sphingolipid, a glycosylglyceride, a cerebroside, or the like), a prostaglandin, an isoprenoid, a terpene, a steroid, a polyphenol, catechin, and a vitamin (B1, B2, B3, B5, B6, B7, B9, B12, C, A, D, E, or the like). Existing components or candidate components such as a pharmaceutical drug, a nutritional food product, a supplement, a food additive, a pesticide, and perfumery (a cosmetic) are also one of the preferable test substances. A plant extract, a cell extract, a culture supernatant or the like may be used as the test substance. By adding two or more therapeutic drugs or test substances at the same time, the interaction, the synergism, or the like between the therapeutic drugs or the test substances may be examined. The therapeutic drug or the test substance may be of natural origin or synthetic. In the latter case, an efficient assay system can be constructed using, for example, a combinatorial synthesis technique.

The period for bringing the therapeutic drug or the test substance into contact can be appropriately set. The contact period is, for example, 10 minutes to 3 days and preferably 1 hour to 1 day. The contact may be carried out a plurality of times.

In a first aspect of the step (iii), the therapeutic drug or the test substance that has permeated through the cell layer is quantified. For example, in a case where a culture container equipped with a cell culture insert such as Transwell (registered trade name) is used, a therapeutic drug or test substance that has permeated through the cell culture insert, that is, the therapeutic drug or test substance that has moved into the upper or lower vessel via the cell layer is quantified depending on the therapeutic drug or test substance by a measuring method such as mass spectrometry, liquid chromatography, and immunological methods (for example, a fluorescent immunoassay method (an FIA method) and an enzymatic immunoassay method (an EIA method)). Based on the quantification results (the amount of the test substance that has permeated through the cell layer) and the amount of the test substance used (typically, the amount added to the culture medium), the absorbability or membrane permeability, drug interaction, induction of a drug metabolizing enzyme, induction of a drug transporter, immune response, or toxicity are determined and evaluated with respect to the therapeutic drug or the test substance.

Alternatively, in order to evaluate the absorption of the therapeutic drug or the test substance, for example, the residual amount of the therapeutic drug or the test substance in the culture solution may be measured. A suitable measuring method may be selected depending on the test substance. For example, mass spectrometry, liquid chromatography, and an immunological method (for example, a fluorescent immunoassay method (FIA method) and an enzymatic immunoassay method (EIA method), or the like can be employed. Typically, in a case where a decrease in the content of the therapeutic drug or the test substance in the culture solution is recognized, it is determined or evaluated that "the therapeutic drug or the test substance has been absorbed". In addition, the absorption amount or the absorption efficiency of the therapeutic drug or the test substance can be determined or evaluated depending on the degree of the decrease. In addition, the absorption can also be evaluated by measuring the amount of the therapeutic drug or test substance incorporated into the cells.

In a second aspect of the step (iii), a hormone that is secreted by the enteroendocrine cells is quantified. The hormone that is secreted by the enteroendocrine cells can be quantified by a measuring method such as mass spectrometry, liquid chromatography, an immunological method (for example, a fluorescent immunoassay method (FIA method), or an enzymatic immunoassay method (EIA method)).

In another aspect according to the present invention, the metabolism of a therapeutic drug or a test substance can be evaluated. In the above-described method the following steps are carried out: (I) a step of bringing a therapeutic drug or a test substance into contact with the enteroendocrine cells; and a step (II) of measuring and evaluating the metabolism of the therapeutic drug or the test substance.

The step (I), that is, bringing of a test substance into contact with enteroendocrine cells or a therapeutic drug can be carried out in the same manner as in the step (ii). However, it is not essential to form a cell layer in advance.

After the step (I), the metabolism of the therapeutic drug or the test substance is measured and evaluated (the step (II)). The metabolism may be measured and evaluated, substantially without time interval, immediately after the step (I), that is, after bringing the therapeutic drug or the test substance into contact with cells, or the metabolism may be measured and evaluated after a certain time (for example, 10 minutes to 5 hours) has passed. The measurement of metabolism can be carried out, for example, by detecting a metabolite. In this case, the expected metabolite is usually qualitatively or quantitatively measured using the culture solution after the step (I) as a sample. A suitable measuring method may be selected depending on the metabolite, and for example, mass spectrometry, liquid chromatography, and an immunological method (for example, a fluorescent immunoassay method (FIA method) and an enzymatic immunoassay method (EIA method)), or the like can be employed.

Typically, in a case where a metabolite of a therapeutic drug or a test substance is detected, it is determined or evaluated that "the therapeutic drug or the test substance has been metabolized". In addition, the metabolism quantity of the therapeutic drug or the test substance can be evaluated depending on the amount of the metabolite. The metabolism efficiency of the therapeutic drug or the test substance may be calculated based on the detection result of the metabolite and the used amount of the therapeutic drug or the test substance (typically, the amount added to the culture medium).

The metabolism of the therapeutic drug or the test substance can be measured using, as an indicator, the expression of drug metabolizing enzymes (cytochrome P450 (particularly CYP3A4), uridine diphosphate glucuronosyltransferase (particularly UGT1A8 or UGT1A10), and sulfotransferase (particularly SULT1A3 or the like)) in enteroendocrine cells. The expression of drug metabolizing enzymes can be evaluated at the mRNA level or the protein level. For example, in a case where an increase in the mRNA level of a drug metabolizing enzyme is recognized, it can be determined that "the therapeutic drug or the test substance has been metabolized". Similarly, in a case where an increase in the activity of a drug metabolizing enzyme is recognized, it can be determined that "the therapeutic drug or the test substance has been metabolized". Similarly to the case where the metabolite is used as an indicator for determination, quantitative determination or evaluation may be carried out based on the expression level of the drug metabolizing enzyme.

In addition, the measurement or evaluation of the metabolism and the measurement or evaluation of the absorbance may be carried out simultaneously or in parallel.

As a still another use application of the enteroendocrine cells, a cell preparation containing the enteroendocrine cells is provided. The cell preparation can be applied to the treatment of various intestinal diseases. In particular, use as a material for regeneration/reconstruction of a damaged intestinal epithelial tissue (including dysfunction) is considered. That is, the contribution to regenerative medicine can be expected. The cell preparation can be prepared by, for example, suspending the enteroendocrine cells in saline or a buffer solution (for example, a phosphate buffer solution) or producing a three-dimensional tissue (organoid or spheroid) using the intestinal epithelial cell-like cells. In order to be able to administer a therapeutically effective amount of cells, a single dose may contain, for example, 1 × 10⁵ to 1 × 10¹⁰ cells. The cell content can be suitably adjusted in consideration of the purpose of use, the target disease, the gender, age, weight, and state of the affected part of the target (recipient) to be administered, cell state.

Dimethyl sulfoxide (DMSO) and serum albumin for the protection of cells, an antibiotic for the prevention of bacterial contamination, and various components (a vitamin, a cytokine, a growth factor, a steroid, and the like) for the activation, proliferation, induction of differentiation, or the like of cells may be contained in the cell preparation according to the embodiment of the present invention. In addition, other pharmaceutically acceptable components (for example, a carrier, an excipient, a disintegrating agent, a buffering agent, an emulsifying agent, a suspending agent, a soothing agent, a stabilizer, a preservative, an antiseptic agent, saline, and the like) may be contained in the cell preparation.

Examples of the still another use application of the enteroendocrine cells include culturing a microorganism (a bacterium, a fungus, a virus, or the like) or a parasite by using the enteroendocrine cells. In a case where a microorganism or a parasite is cultured using the enteroendocrine cells, it is possible to analyze the infectivity of the microorganism or the parasite to the enteroendocrine cells. In addition, in a case where the infectivity is present, it is possible to check the maintenance or proliferation ability of the microorganism or the parasite. In addition, in a case of culturing a microorganism or a parasite using the enteroendocrine cells, it is possible to obtain a microorganism or a parasite, which proliferates repeatedly. Further, it is possible to reproduce and utilize the infectious state of a pathogenic microorganism for the screening of a therapeutic drug or reproduce the effect of a substance produced by a microorganism or a parasite on the intestinal cells to screen for substances useful or harmful to the small intestine of a living body.

Specific examples of the microorganism or parasite include Escherichia coli, a lactic acid bacterium, a bifidus bacterium, an enteritis vibrio bacterium, a salmonella bacterium, various intestinal bacteria, an influenza virus, a rotavirus, an adenovirus, an astrovirus, a Sapporo virus, norovirus, an HIV, and a coronavirus, which are not limited thereto.

Examples of the still another use application of the enteroendocrine cells include a co-culture model using the enteroendocrine cells. According to the co-culture model described above, it is possible reproduce the structure of the biological tissue and analyze the interaction with other cells. In addition, according to the co-culture model described above, it is possible to reproduce the pathological condition of a disease and carry out screening or evaluation for a therapeutic drug or a test substance.

The cell to be co-cultured is a cell species that is present in a living body of a primate or a rodent, and it is particularly preferably a cell that is present in the vicinity of the small intestine. Examples thereof include, but are not limited to, a hepatocyte, pancreas, a vascular endothelial cell, an immune cell, nerve, a fibroblast, a muscle cell, and a mesenchymal cell. The cell to be co-cultured may be a primate or rodent-derived established cell line. Examples thereof include, but are not limited to, an immortalized cell line such as a Hela cell and a THP-1 cell, a pluripotent stem cell such as an ES cell or an iPS cell, and a cell differentiated from the pluripotent stem cells.

As the therapeutic drug or the test substance that can be tested in the co-culture model, organic compounds or inorganic compounds having various molecular sizes can be used. Existing components or candidate components such as medicines and nutritional food products are also preferred test substances. A plant extract, a cell extract, a culture supernatant or the like may be used as the test substance. In addition, two or more kinds of these may be used in combination.

### <Culture medium or culture medium kit, and kit for producing enteroendocrine cell>

According to the present invention, there is provided a culture medium or a culture medium kit, containing a basal medium, serum or a serum substitute, a cAMP activator, a DNA methyltransferase inhibitor, a transforming growth factor-β signal inhibitor, and a MEK inhibitor, where the culture medium or the culture medium kit substantially does not contain a ligand of an epidermal growth factor. The culture medium or the culture medium kit according to the embodiment of the present invention may further contain a Notch inhibitor. The culture medium or the culture medium kit according to the embodiment of the present invention can be used for producing enteroendocrine cells.

The details of the basal medium, the serum or serum substitute, the cAMP activator, the DNA methyltransferase inhibitor, the transforming growth factor-β signal inhibitor, the MEK inhibitor, the ligand of an epidermal growth factor, and the Notch inhibitor are as described above in the present specification.

According to the present invention, there is further provided a kit for producing an enteroendocrine cell, the kit containing the above-described culture medium or culture medium kit according to the embodiment of the present invention, and an intestinal stem cell or an intestinal epithelial cell, which is induced from a pluripotent stem cell. The details of the intestinal stem cell or the intestinal epithelial cell, which has been induced from a pluripotent stem cell, are as described above in the present specification.

The present invention will be further specifically described with reference to Examples; however, the present invention is not limited by Examples.

### Examples

### Example 1

### <Method>

Human iPS cell-derived intestinal epithelial cells (including intestinal stem cells, LGR5+ cells) F-hiSIEC^{™} (FUJIFILM Corporation) were thawed on the day 0 and seeded at 1.0 × 10⁶ cells/well on a cell culture insert coated with Matrigel^{™} (Corning Incorporated) by using F-hiSIEC^{™} Seeding Medium (FUJIFILM Corporation), and then from the next day, the culture medium was exchanged every other day with each culture medium (culture medium compositions 1 to 6) in Table 1. Coating, seeding, and culture medium exchange were carried out according to the F-hiSIEC instruction manual to obtain a cell sheet. The total RNA was extracted from the cells on the Day 9. For RNA extraction, the cells were lysed with Qiazol (QIAGEN), 20% of chloroform was added thereto, mixed, and centrifuged at 12,000 g for 10 minutes to obtain a solution, and the supernatant of the solution was recovered. Total RNA was extracted from the supernatant according to the manual using an RNeasy mini kit (QIAGEN). cDNA was synthesized from the total RNA using a High Capacity RNA-to-cDNA kit (Applied Biosystems) according to the manual. The obtained cDNA was subjected to the measurement of the gene expression level by using a ViiA7 real-time PCR system (Applied Biosystems) with a Taqman Gene Expression Assay system and a Taqman Gene Expression Master Mix (Applied Biosystems). The Taqman Assay ID used were as follows: 18s: 03003631_g1, CHGA: Hs00900370_m1, TPH1: Hs00188220_m1, PYY: Hs00373890_g1, and REG4: Hs00230746_m1.

**[Table 1]**

| Culture medium composition | | | 1 | 2 | 3 | 4 | 5 | 6 |
|---|---|---|---|---|---|---|---|---|
| Culture medium | DMEM/F12 | | + | + | + | + | + | + |
| | B27 | 2% | | | | | | |
| | HepEx | 1% | | | | | | |
| Fetal bovine serum | FBS | 2% | | | | | | |
| cAMP activator | Forskolin | 30µmol/L | | | | | | |
| MEK inhibitor | PD98059 | 20µmol/L | | | | | | |
| TGFβ signal inhibitor | A83-01 | 0.5µmol/L | | | | | | |
| DNMT inhibitor | 5-Aza-2'-deoxycytidine | 5µmol/L | | | | | | |
| Growth factor | EGF | 0.02µg/mL | + | + | - | - | - | - |
| Notch inhibitor | LY41157 | 1µmol/L | - | + | - | + | + | - |
| | DAPT | 10µmol/L | - | - | - | - | - | + |
| Wnt inhibitor | IWP2 | 5µmol/L | - | - | - | - | + | - |

Gene expression levels for comparison are indicated in terms of relative value in a case where the expression in the control group cultured in the F-hiSIEC Culture Medium is corrected with the expression level of the internal standard gene 18S according to the ΔΔCT method and then is set to 1.

### <Result>

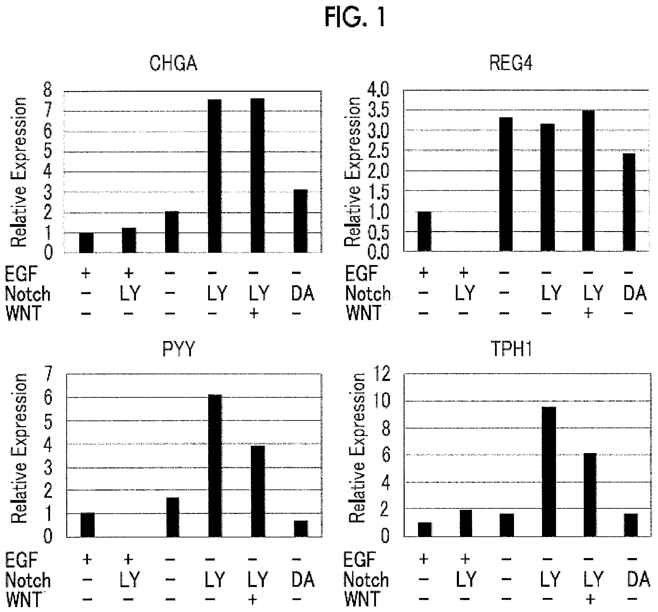

The results are shown in Fig. 1.

As compared with the gene expression in the culture medium composition 1 for culturing normal intestinal epithelial cells, there was no significant change in the expression of CHGA, which is an EEC marker in the culture medium composition 2 in which a Notch inhibitor was added in the presence of EGF and the culture medium composition 3 in which EGF was removed. On the other hand, in a case where EGF was removed and a Notch inhibitor was added, a marked increase in the expressions of CHGA and REG4, which are EEC markers, was observed. Further, an expression of the EEC marker equal to or larger than the expression in the culture medium composition 4 was not observed in the culture medium composition 5 obtained by adding a Wnt inhibitor to the culture medium composition 4. In addition, in a case where LY411575 (culture medium composition 4) and DAPT (culture medium composition 6) were compared among Notch inhibitors, in the composition 4 in which LY411575 was used, not only the expression of CHGA and Reg4, which are EEC markers, but also the expression of TPH1 or PYY, which is a factor involved in the hormone secretion which is a function of enteroendocrine cells were remarkably high.

### Example 2

### <Method>

Human iPS cell-derived intestinal epithelial cells (including intestinal stem cells, LGR5+ cells) F-hiSIEC^{™} (FUJIFILM Corporation) were thawed on the day 0 and seeded at 1.0 × 10⁶ cells/well on a cell culture insert coated with Matrigel^{™} (Corning Incorporated) by using F-hiSIEC^{™} Seeding Medium (FUJIFILM Corporation), and then from the next day, the culture medium was exchanged every other day with each culture medium (culture medium compositions 7 to 13) in Table 2. Coating, seeding, and culture medium exchange were carried out according to the F-hiSIEC instruction manual to obtain a cell sheet. The total RNA was extracted from the cells on the Day 9. For RNA extraction, the cells were lysed with Qiazol (QIAGEN), 20% of chloroform was added thereto, mixed, and centrifuged at 12,000 g for 10 minutes to obtain a solution, and the supernatant of the solution was recovered. Total RNA was extracted from the supernatant according to the manual using an RNeasy mini kit (QIAGEN). cDNA was synthesized from the total RNA using a High Capacity RNA-to-cDNA kit (Applied Biosystems) according to the manual. The obtained cDNA was subjected to the measurement of the gene expression level by using a ViiA7 real-time PCR system (Applied Biosystems) with a Taqman Gene Expression Assay system and a Taqman Gene Expression Master Mix (Applied Biosystems). The Taqman Assay ID used were as follows: 18s: 03003631_g1, CHGA: Hs00900370_m1, PYY: Hs00373890_g1, and NEUROG3: Hs01875204_s1.

**[Table 2]**

| Culture medium composition | | 7 | 8 | 9 | 10 | 11 | 12 | 13 |
|---|---|---|---|---|---|---|---|---|
| Culture medium | DMEM/F12 | + | + | + | + | + | + | + |
| | B27 2% | | | | | | | |
| | HepEx 1% | | | | | | | |
| Fetal bovine serum | FBS 2% | | | | | | | |
| cAMP activator | Forskolin | | | | | | | |
| | 30µmol/L | | | | | | | |
| MEK inhibitor | PD98059 | | | | | | | |
| | 20µmol/L | | | | | | | |
| TGFβ signal inhibitor | A83-01 | | | | | | | |
| | 0.5µmol/L | | | | | | | |
| DNMT inhibitor | 5-Aza-2'-deoxycytidine | | | | | | | |
| | 5µmol/L | | | | | | | |
| Growth factor | EGF | - | - | - | - | - | - | - |
| Notch inhibitor | LY41157 (µmol/L) | - | 0.5 | 0.75 | 1.0 | 2.0 | - | - |
| | DAPT (µmol/L) | - | - | - | - | - | 5.0 | 10 |

Gene expression levels for comparison are indicated in terms of relative value in a case where the expression in the control group cultured in the F-hiSIEC Culture Medium is corrected with the expression level of the internal standard gene 18S according to the ΔΔCT method and then is set to 1.

### <Result>

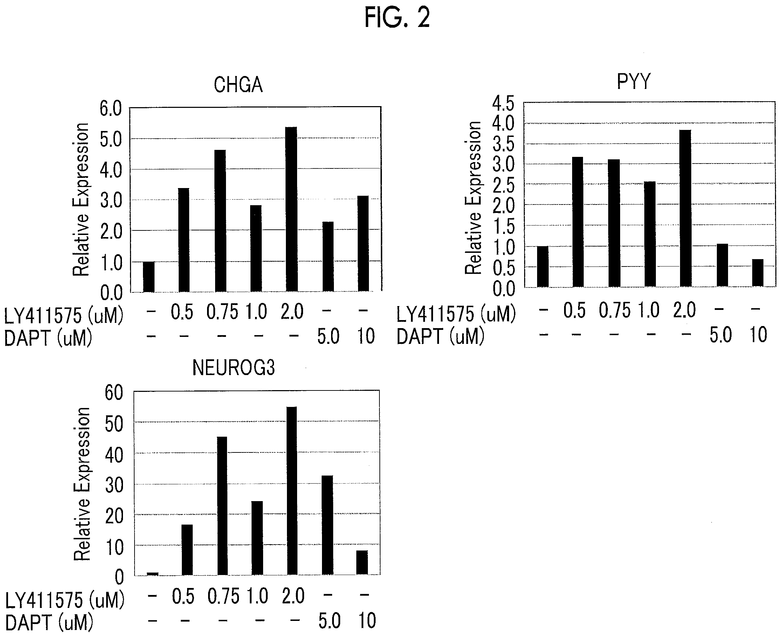

The results are shown in Fig. 2.

As compared with the DAPT addition groups (culture medium compositions 8 to 11), in the LY411575 addition groups (culture medium compositions 12 and 13), the expression of CHGA, which an EEC marker, was high, and further, the expression of PYY, which is a factor involved in the hormone secretion which is a function of enteroendocrine cells was remarkably high. In addition, the expression levels of CHGA, PYY, and NEUROG3 were highest in the LY411575 2 µmol/L addition group (the culture medium composition 11).

### Example 3

### <Method>

Human iPS cell-derived intestinal epithelial cells (including intestinal stem cells, LGR5+ cells) F-hiSIEC^{™} (FUJIFILM Corporation) were thawed on the day 0 and seeded at 1.0 × 10⁶ cells/well on a cell culture insert coated with Matrigel^{™} (Corning Incorporated) by using F-hiSIEC^{™} Seeding Medium (FUJIFILM Corporation), and then from the next day, the culture medium was exchanged every other day with the culture medium in Table 3. The obtained cells were denoted as EEC. In addition, cells obtained by being subjected to the culture medium change with the F-hiSIEC Culture Medium according to the same schedule were used as a control. Coating, seeding, and culture medium exchange were carried out according to the F-hiSIEC instruction manual to obtain a cell sheet. Total RNA was extracted from the cells on the Day 9. In addition, a human colon adenocarcinoma cell line Caco-2 was purchased from KAC Co., Ltd. and cultured in a culture medium (MEM E, 1% NEAA, 10% FBS) according to the manual. For RNA extraction, the cells were lysed with Qiazol (QIAGEN), 20% of chloroform was added thereto, mixed, and centrifuged at 12,000 g for 10 minutes to obtain a solution, and the supernatant of the solution was recovered. Total RNA was extracted from the supernatant according to the manual using an RNeasy mini kit (QIAGEN). cDNA was synthesized from the total RNA using a High Capacity RNA-to-cDNA kit (Applied Biosystems) according to the manual. The obtained cDNA was subjected to the measurement of the gene expression level by using a ViiA7 real-time PCR system (Applied Biosystems) with a Taqman Gene Expression Assay system and a Taqman Gene Expression Master Mix (Applied Biosystems). In the Taqman assay, 18s: 03003631_g1 and NKX6.1: Hs00232355_m1 were used. Gene expression levels for comparison are indicated in terms of relative value in a case where the expression in Caco-2 is corrected with the expression level of the internal standard gene 18S according to the ΔΔCT method and then is set to 1.

**[Table 3]**

| Added factor | | |
|---|---|---|
| Culture medium | DMEM/F12 | |
| | B27 | 2% |
| | HepEx | 1% |
| Fetal bovine serum | FBS | 2% |
| cAMP activator | Forskolin | 30µmol/L |
| MEK inhibitor | PD98059 | 20µmol/L |
| TGFβ signal inhibitor | A83-01 | 0.5µmol/L |
| DNMT inhibitor | 5-Aza-2'-deoxycytidine | 5µmol/L |
| Growth factor | EGF | - |
| Notch inhibitor | LY41157 | 1µmol/L |

### <Result>

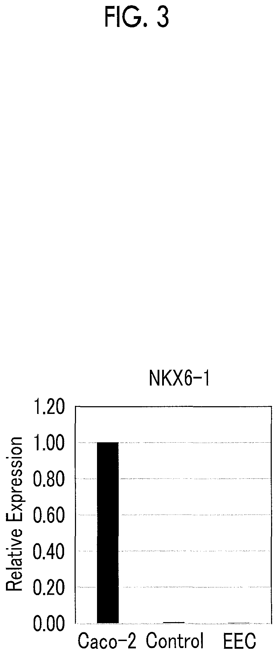

The results are shown in Fig. 3.

In a case where the expression of NKX6.1 in Caco-2 was set to 1, the expression of NKX6.1 was not observed in the control and the cells obtained by being cultured using the culture medium of Table 3.

## Claims

1. A method of producing an enteroendocrine cell, the method comprising:
culturing an intestinal stem cell or an intestinal epithelial cell, which is induced from a pluripotent stem cell, by using a culture medium that substantially does not contain a ligand of an epidermal growth factor but contains a cAMP activator and a DNA methyltransferase inhibitor.

2. The method according to claim 1,
wherein the cAMP activator is forskolin, and
the DNA methyltransferase inhibitor is 5-aza-2'-deoxycytidine.

3. The method according to claim 1 or 2,
wherein the culture medium contains any one or more of a transforming growth factor-β signal inhibitor and a MEK inhibitor.

4. The method according to claim 3,
wherein the transforming growth factor-β signal inhibitor is A83-01, and
the MEK inhibitor is PD98059 and/or PD0325901.

5. The method according to claim 1 or 2,
wherein the culture medium contains a Notch inhibitor.

6. The method according to claim 5,
wherein the Notch inhibitor is LY411575.

7. The method according to claim 1 or 2,
wherein the culture medium does not contain any one or more of a Wnt activator and a Wnt inhibitor.

8. The method according to claim 1 or 2,
wherein the culture medium does not contain any one or more of a retinoic acid signal pathway activator and a Sonic hedgehog signal pathway inhibitor.

9. The method according to claim 1 or 2,
wherein the pluripotent stem cell is a human induced pluripotent stem cell.

10. The method according to claim 1 or 2,
wherein the culture step using the culture medium does not include a suspension culture step.

11. The method according to claim 1 or 2,
wherein spheroids are not formed in the culture step using the culture medium.

12. The method according to claim 1 or 2,
wherein the enteroendocrine cell to be produced has a sheet shape.

13. A method of analyzing a disease mechanism, the method comprising:
a step of producing an enteroendocrine cell by the method according to claim 1 or 2; and
a step of analyzing a disease mechanism by using the enteroendocrine cell.

14. An evaluation method for a test substance, comprising:
a step of producing an enteroendocrine cell by the method according to claim 1 or 2; and
a step of quantifying a hormone that is secreted by the enteroendocrine cell in a presence of a test substance.

15. A screening method for a therapeutic drug for a digestive system disease, the screening method comprising:
a step of producing an enteroendocrine cell by the method according to claim 1 or 2; and
a step of culturing the enteroendocrine cell in a presence of a digestive system disease.

16. An enteroendocrine cell derived from a pluripotent stem cell,
wherein the enteroendocrine cell expresses TPH1, PYY, CHGA, REG4, and NEUROG3 but does not express NKX6.1.

17. A culture medium or a culture medium kit, comprising:
a basal medium;
serum or a serum substitute;
a cAMP activator;
a DNA methyltransferase inhibitor;
a transforming growth factor-β signal inhibitor; and
a MEK inhibitor,
wherein the culture medium or the culture medium kit substantially does not contain a ligand of an epidermal growth factor.

18. The culture medium or the culture medium kit according to claim 17, further comprising:
a Notch inhibitor.

19. The culture medium or the culture medium kit according to claim 17 or 18,
wherein the culture medium or the culture medium kit is for producing an enteroendocrine cell.

20. A kit for producing an enteroendocrine cell, the kit comprising:
the culture medium or the culture medium kit according to claim 17 or 18; and
an intestinal stem cell or an intestinal epithelial cell, which is induced from a pluripotent stem cell.
